(19) **Europäisches Patentamt**

**European Patent Office**

**Office européen des brevets**

(11) **EP 0 476 923 B1**

(12) **EUROPEAN PATENT SPECIFICATION**

(45) Date of publication and mention
of the grant of the patent:
**31.01.1996  Bulletin 1996/05**

(51) Int Cl.6: **G01N 33/49**

(21) Application number: **91308303.6**

(22) Date of filing: **11.09.1991**

(54) **Blood coagulation time measurement**

Blutgezinnungszeitmessung

Mesure du temps de coagulation du sang

(84) Designated Contracting States:
**AT BE CH DE DK ES FR GB GR IT LI LU NL SE**

(30) Priority: **14.09.1990 JP 245825/90**

(43) Date of publication of application:
**25.03.1992  Bulletin 1992/13**

(73) Proprietor: **Sankyo Company Limited
Tokyo (JP)**

(72) Inventors:
• **SAITO, Yukio,
c/o Biological Research Laboratory
Shinagawa-ku, Tokyo (JP)**
• **SEKIYA, Kochi,
c/o Biological Research Laboratory
Shinagawa-ku, Tokyo (JP)**

(74) Representative: **Bibby, William Mark et al
Ickenham, Uxbridge UB10 8BZ (GB)**

(56) References cited:
**EP-A- 0 332 110          GB-A- 2 005 014
US-A- 3 821 643**

## Description

BACKGROUND OF THE INVENTION

1. Field of the Invention

The present invention relates to an apparatus for measuring a blood coagulation time. The apparatus according to the present invention is used for measuring the coagulation time of blood, such as ordinary blood, whole blood, hyperlipemia blood plasma, and blood plasma with high hemolysis, etc., including optically opaque blood, blood having an extended coagulation time, and blood having a low coagulation function.

2. Description of the Related Arts

In general, the measurement of the blood coagulation time is conducted for the thrombo test (TB), the hepa test (HP), the prothrombin time (PT), the activated partial thromboplastin time (APTT), and the fibrinogen concentration (FIB), etc.

In the measurement of the blood coagulation time, a trigger reagent is added to a blood plasma sample in a test tube or a cylindrical vessel, to start a coagulation reaction. Then, by utilizing the phenomenon that the fibrinogen is changed into a fibrin according to the coagulation reaction, the physical properties such as fluidity, viscosity, and optical turbidity, etc. are monitored, and based on the change of these physical properties, the time of the end of the coagulation process is detected, whereby the time from the addition of the trigger reagent to the end of the coagulation process is measured as the blood coagulation time.

In the prior arts, a number of methods, such as the manual method, the mechanical method, and the optical method, have been used for monitoring the physical properties in the coagulation process.

In the manual method, the fluidity of the sample in a test tube, in the inclined state when held in the hand, is observed to determine the process of coagulation and monitor the change of the fluidity, and based on the observation of the change of the fluidity, the time of the end of the coagulation process is determined.

In the mechanical method, a small steel ball is contained in a reaction vessel in which an amount of a blood plasma sample is to be supplied, and an external magnetic sensor connected to a detection circuit is placed outside the reaction vessel corresponding to the steel ball. The longitudinal axis of the reaction vessel is inclined from the vertical direction, and the reaction vessel is rotated by a drive device.

When only an amount of a blood plasma sample is supplied in the rotating reaction vessel, the steel ball rotates in the blood sample at the bottom of the reaction vessel but holds an approximately fixed position relative to the external magnetic sensor, due to a high fluidity of the blood plasma sample. This fixed state of the steel ball is detected by the detection circuit via the magnetic sensor.

When a coagulation reagent is added to the blood plasma sample and a coagulation of the blood plasma sample is attained, the steel ball moves together with the rotating motion of the reaction vessel, due to a low fluidity of the blood plasma sample, and the distance between the steel ball and the magnetic sensor is periodically changed. This change of the distance between the steel ball and the external magnetic sensor is detected by the detection circuit via the magnetic sensor, and thus the time of the end of the coagulation of the blood plasma sample can be determined.

In the optical method, a light source and a plurality of optical sensors connected to detection circuits are arranged outside the reaction vessel in which an amount of a blood plasma sample is to be supplied, and a light is irradiated onto the blood plasma sample in the reaction vessel.

When only an amount of a blood plasma sample is supplied to the reaction vessel, the output of the optical sensor, which represents the amount of scattered light reflected by the blood plasma sample, becomes lower than a predetermined value due to an optical property of the blood plasma sample.

When a coagulation reagent is added to the blood plasma sample and the coagulation of the blood sample is attained, the output of the optical sensor, which represents the amount of scattered light reflected by the blood plasma sample, is remarkably increased in accordance with changes in the optical property of the blood plasma sample due to the coagulation of the blood plasma sample, and thus the time of the end of the coagulation of the blood plasma sample can be determined.

In the prior art manual method, however, the handling is difficult to some extent, and it is difficult to correctly determine the time of the end of the coagulation process.

Also, in the prior art mechanical method, although the detection of the time of the end of the coagulation process for a blood plasma sample having a normal coagulation nature is possible, it is difficult to stably determine the time of the end of the coagulation process for an abnormal blood plasma sample having an extended coagulation time, particularly a blood plasma sample having a low coagulation nature. Accordingly, it is difficult to continuously monitor the coagulation process, and thus it is difficult to conduct a detailed analysis of the coagulation nature of the blood plasma sample.

Also, in the prior art optical method, although the detection of the time of the end of the coagulation process for an ordinary blood plasma sample is possible, it is difficult to determine the time of the end of the coagulation process for optically opaque samples, such as whole blood, hyperlipemia blood plasma, and highly hemolytic blood plasma, etc.

A prior art blood coagulation timer is described, for example, in US-A-3821643, and a prior art optical method is described, for example in GB-A-2005014. However, further improvements in enhancing the sensitivity of detection of the time of the end of the blood coagulation have been sought.

An object of the present invention is to provide an improved apparatus for measuring a blood coagulation time in which the time of the end of the coagulation process can be determined not only for an ordinary blood plasma but also for optically opaque samples, such as whole blood, hyperlipemia blood plasma, and highly hemolytic blood plasma, etc.

Another object of the present invention is to provide an improved apparatus for measuring a blood coagulation time in which the time of the end of the coagulation process for an abnormal blood plasma sample having an extended coagulation time, particularly a blood plasma sample having a low coagulation nature, can be stably determined.

Another object of the present invention is to provide an improved apparatus for measuring a blood coagulation time in which the time of the end of the coagulation time can be continuously monitored.

Therefore, in accordance with the present invention, there is provided an apparatus for measuring a blood coagulation time by a measurement of a period from a time of an addition of a coagulation reagent to a sample of blood, such as blood plasma and whole blood, to a time of a coagulation of the sample, the apparatus including a vessel for holding a sample of blood, such as blood plasma and whole blood, a semiconductor sensor having a negative temperature coefficient and being immersed in the sample of blood held in the vessel; an electric power source connected to the semiconductor sensor; a positive self-feedback resistor connected in association with the power source for increasing the sensitivity of detection; and a detection circuit connected to the output terminals of the semiconductor sensor for detecting the output signal of the semconductor sensor.

BRIEF DESCRIPTION OF THE DRAWINGS

Figure 1 shows an apparatus for measuring a blood coagulation time according to an embodiment of the present invention;
Fig. 2 illustrates an example of the characteristic of the operation of the apparatus of Fig. 1;
Fig. 3 shows an apparatus for measuring a blood coagulation time according to another embodiment of the present invention;
Fig. 4 shows an example of the structure of the voltage detection portion of an apparatus for measuring a blood coagulation time according to an embodiment of the present invention;
Figs. 5 and 6 shows apparatuses for measuring a blood coagulation time according to other embodiments of the present invention;
Fig. 7 shows an apparatus for measuring a blood coagulation time according to another embodiment of the present invention;
Fig. 8'illustrates an example of the characteristic of the operation of the apparatus of Fig. 7;
Fig. 9 illustrate an example of the manner of detection of blood coagulation end point;
Fig. 10 illustrates a characteristic of the relationship between the temperature of the semiconductor sensor and the power consumption;
Fig. 11 illustrates a characteristic of the coagulation process, represented by the change of temperature;
Fig. 12 illustrates a characteristic of the coagulation process, represented a change with time of the once-differentiated temperature;
Fig. 13 illustrates a characteristic of the coagulation process, represented by a change with time of the power consumption;
Fig. 14 illustrates a characteristic of the coagulation process, represented by a change with time of the thermal resistance;
Figs. 15 to 17 illustrate the PT characteristics for whole blood;
Figs. 18 to 20 illustrate the PT characteristics for diluted whole blood of 6.25%;
Figs. 21 to 23 illustrate the PT characteristics for blood plasma;
Figs. 24 to 26 illustrate the PT characteristics for diluted blood plasma of 6.25%;
Fig. 27 illustrates a characteristic of the PT activity;
Fig. 28 illustrates a characteristic of the APTT activity;
Fig. 29 illustrates a characteristic of the FIB concentration;
Fig. 30 illustrates a characteristic of the HP activity; and
Fig. 31 illustrates a characteristic of the TB activity.

DESCRIPTION OF THE PREFERRED EMBODIMENTS

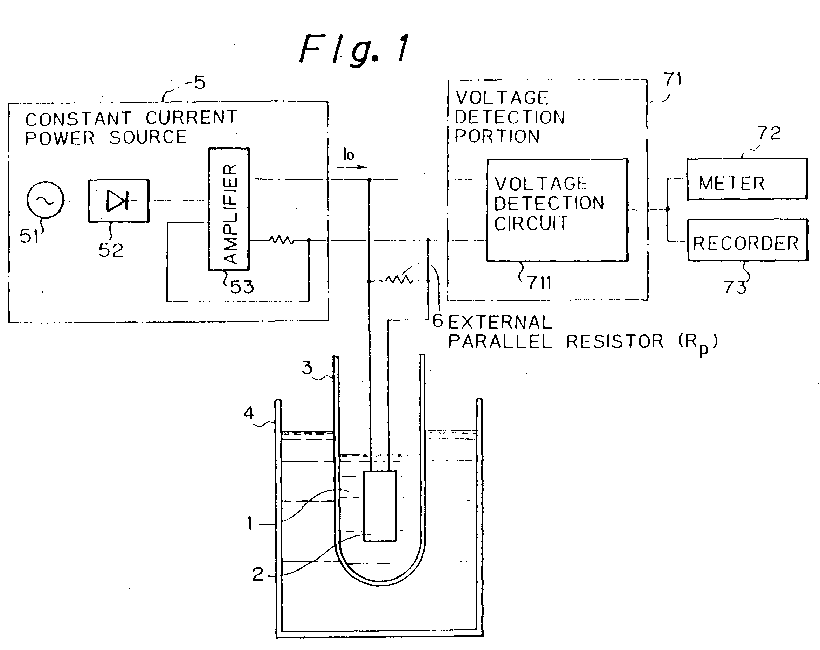

An apparatus for measuring a blood coagulation time according to an embodiment of the present invention is shown in Fig. 1. The apparatus of Fig. 1 is constituted by a blood sample 1 and a thermistor having a negative temperature coefficient of resistance 2 as a semiconductor sensor contained in a reaction vessel 3, a constant temperature bath 4 accommodating the reaction vessel 3, a constant current power source 5 having an alternate current source 51, a rectifier 52, and an amplifier 53 with a feedback circuit and delivering a constant current $I_o$, an external resistor 6 in a parallel arrangement and having a resistance $R_p$, a voltage detection portion 71 having a voltage detection circuit 711, a meter 72, and a recorder 73.

For example, a JOHN FLUK 3330B type constant current power source is used for the constant current power source 5.

The thermistor 2 is driven by the constant current power source 5 via the external resistor 6, which is connected across the output terminals of the constant current power source 5 and in parallel with the thermistor 2.

The temperature of the reaction vessel 3 accommodated in the constant temperature bath 4 is kept at a constant temperature of, for example, 37°C.

The voltage across the terminals of the thermistor 2 is detected by the voltage detection portion 71.

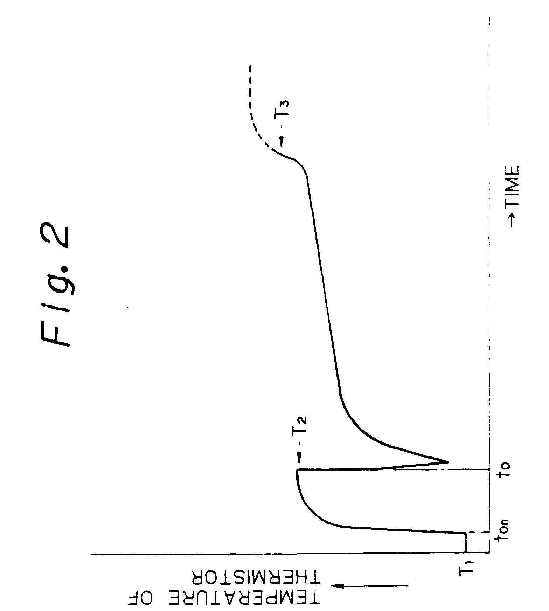

The operation of the apparatus of Fig. 1 will be described with reference to Fig. 2.

A constant current $I_o$ delivered from the constant current power source 5 is supplied to the thermistor 2 immersed in the blood sample 1 in the reaction vessel 3, to heat the thermistor 2. The initial temperature of the thermistor 2 is $T_1$ (°C). The power source is turned on at the time $t_{on}$. After the elapse of a time of, for example 30 secs, a balance of the heat generation and the heat dissipation is attained, and the temperature of the thermistor 2 becomes a fixed value temperature $T_2$ of, for example, 63°C.

The temperature of the thermistor 2 does not mean the temperature of the surface of the thermistor 2 but means the temperature of the inside of the thermistor 2. Since a thermistor is usually coated by glass, metal, or the like for electrical insulation or mechanical reinforcement, the temperature of the surface of a thermistor where a contact with blood sample is formed is lower than the temperature of the inside of the thermistor.

Under this condition, an amount of coagulation reagent of, for example, SANKYO SIMPLASTIN AUTO, is added to the blood sample 1 at the time $t_o$. Although this addition, causes a temporary loss of the heat balance of the substance in the reaction vessel 3, due to a disturbance in the heat diffusion layer around the thermistor 2 caused by the addition of the coagulation reagent, or to the difference between the temperature of the blood sample and that of the added coagulation reagent, the temperature of the substance in the reaction vessel 3 tends to become a new fixed value temperature $T_3$ (°C).

Accordingly, the coagulation reaction of the blood sample 1 proceeds with time, and as the end of the coagulation process approaches, fibrinogen in the blood sample is changed to fibrin to produce clots, and at the same time, the thermal resistivity of the blood sample is increased.

As the thermal resistivity of the blood sample is increased, the quantity of heat radiated from the thermistor 2 is reduced, therefore, the temperature of the thermistor 2 is raised to reach the temperature $T_3$ (°C). Towards the end of the coagulation reaction, if the production of fibrin is terminated, the rate of elevation of the temperature of the thermistor 2 is reduced, and the temperature of the substance in the reaction vessel 3 tends to become a newly fixed value temperature.

As the quantity of heat radiated from the thermistor 2 is changed due to the change of the thermal resistivity of the blood sample, the temperature of the thermistor 2 is changed, and accordingly, the resistance of the thermistor 2 is changed.

As the resistance of the thermistor 2 is changed, the voltage across the terminals of the thermistor 2 is changed. This voltage across the terminals of the thermistor 2 is detected by the voltage detection portion 71.

Therefore, by using the data of the detection of the voltage across the terminals of the thermistor 2, the end of the coagulation process of the blood sample is determined, and by measuring the time from the addition of the coagulation reagent to the end of the coagulation process, the blood coagulation time of the blood sample 1 in the reaction vessel 3 is determined.

In the voltage detection portion 71, it is possible to amplify the input signal, to differentiate the amplified signal, to compare the differentiated amplified signal with a reference signal, and to determine a point where the value of the differentiated amplified signal reaches the value of the reference signal. This point corresponds to the end of the blood coagulation process of the blood sample.

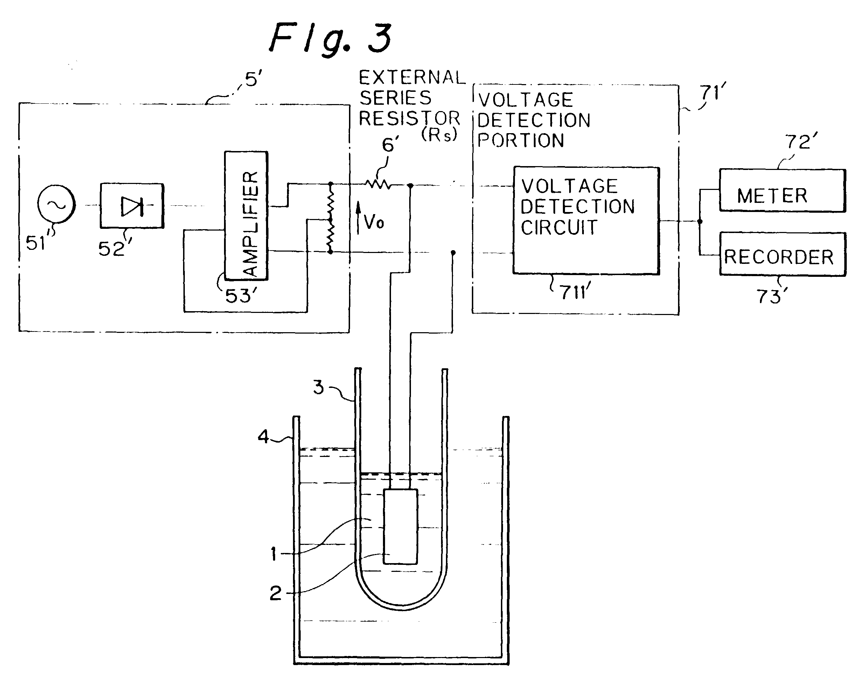

An apparatus for measuring a blood coagulation time according to another embodiment of the present invention is shown in Fig. 3. The apparatus of Fig. 3 is constituted by a blood sample 1 and a thermistor having a negative temperature coefficient of resistance 2 as a semiconductor sensor contained in a reaction vessel 3, a constant temperature bath 4 accommodating the reaction vessel 3, a constant voltage power source 5' having an alternate current source 51', a rectifier 52', and an amplifier 53' with a feedback circuit and delivering a voltage $V_o$, an external resistor 6' in a series arrangement and having a resistance $R_s$, a voltage detection portion 71' having a voltage detection circuit 711', a meter 72', and a recorder 73'.

The operation of the apparatus of Fig. 3 is similar to that of the apparatus of Fig. 1.

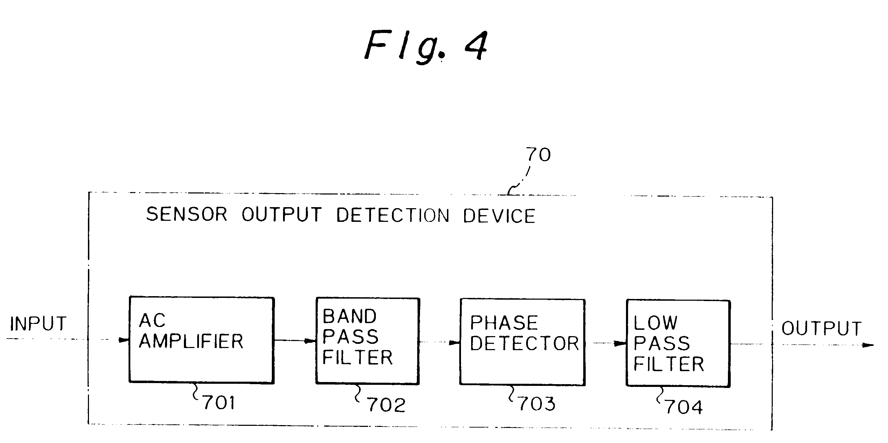

An example of the structure of a sensor output detection device 70 which can be used for an apparatus for measuring a blood coagulation time according to an embodiment of the present invention is shown in Fig. 4. With regard to the case of Fig. 4, an alternate current power source is used for the constant current or constant voltage power source of the apparatus for measuring a blood coagulation time. The device 70 of Fig. 4 is constituted by an alternate current amplifier 701, a band pass filter 702, a phase detector 703, and a low pass filter 704.

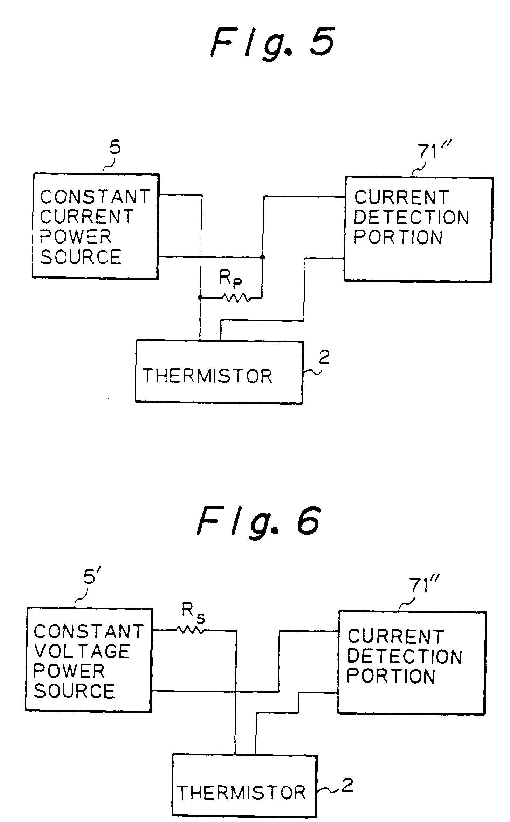

Apparatuses for measuring a blood coagulation time according to other embodiments of the present invention are shown in Fig. 5 and Fig. 6. In the apparatuses of Fig. 5 and Fig. 6, the output of the semiconductor sensor is detected by detecting the direct current flowing through the thermistor 2, by current detection portion 71". The operations of the apparatuses of Fig. 5 and Fig. 6 are similar to the operations of the apparatus of Fig. 1 and Fig. 3.

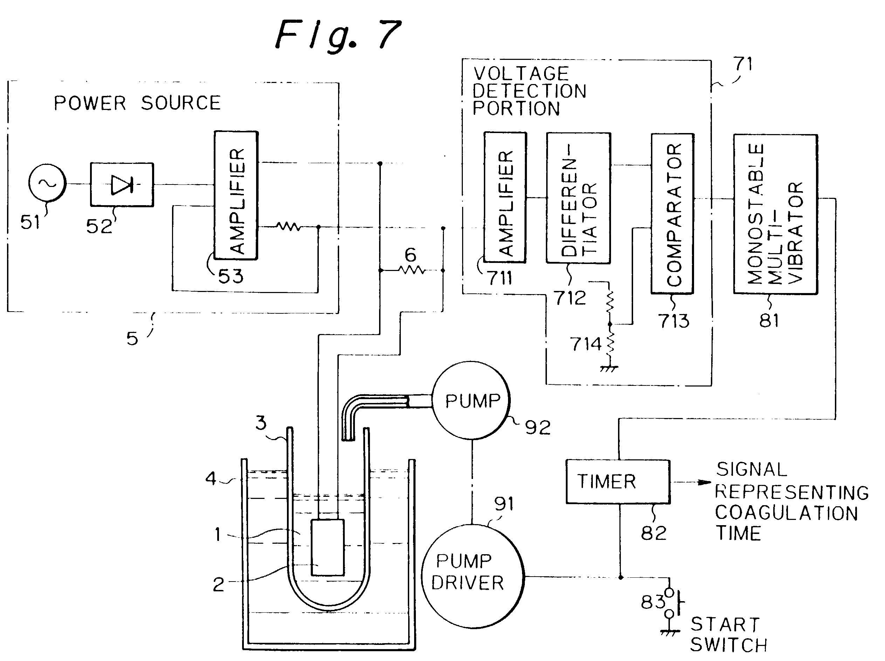

An apparatus for measuring a blood coagulation time according to another embodiment of the present invention is shown in Fig. 7. The apparatus of Fig. 7 is constituted by a blood sample 1 and a thermistor having a negative temperature coefficient of resistance 2 as a semiconductor sensor contained in a reaction vessel 3, a constant temperature bath 4 accommodating the reaction vessel 3, a power source 5, a detection portion 71, a monostable multivibrator 81, a digital counter type or motor type timer 82, a start switch 83, a pump driver 91, and a pump 92 for feeding a coagulation reagent. In the apparatus of Fig. 7, all the processes of adding the coagulation reagent and measuring the coagulation time are fully automatically controlled. Also, it is possible to use a power source having an internal resistor or a power source having an external resistor.

In the voltage detection portion 71, the end of the coagulation process is detected by the detection of the sudden change of the rate of change of the data transformed from the detection data, the rise over a predetermined threshold value of the data transformed from the detection data, and the like.

For this detection, it is possible to use an electronic circuit such as a comparator, or to process the data by an analog-to-digital conversion, and process the converted digital data.

The time count operation of the timer 82 is started when the start switch 83 is turned ON, and is terminated by a signal delivered from the monostable multivibrator 81 indicating a detection of the end of the coagulation process.

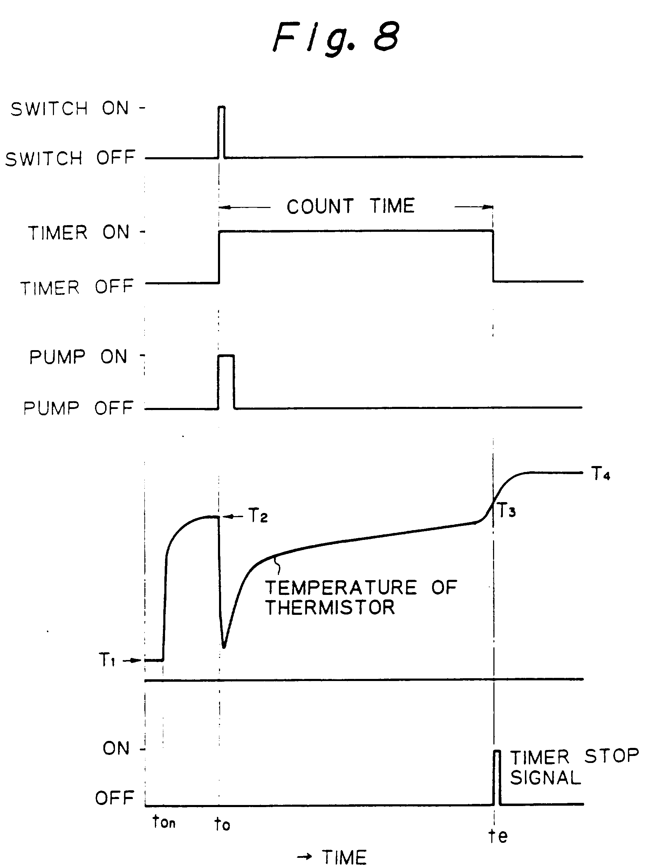

The operation of the apparatus of Fig. 7 will be described with reference to Fig. 8.

A current delivered from the power source 5 flows through the thermistor 2, which is immersed in the blood sample 1 in the reaction vessel 3. When the start switch 83 is turned on at a time $t_o$ , the timer 82 is started, the operation of the pump driver 91 is started, and the operation of the pump 92 is started, and accordingly, an amount of the coagulation trigger reagent is automatically supplied to the reaction vessel 3.

Under this condition, a coagulation reaction is started in the reaction vessel 3 while the heating of the thermistor 2 is continued by the flow of the current from the power source 5 through the thermistor 2.

The voltage across the terminals of the thermistor 2 is detected by the voltage detection portion 71, and the detection of the end of the coagulation process is carried out based on the output of the voltage detection portion 71.

When the end of the coagulation process is detected at the time $t_e$ , a signal is input to the timer 31, to stop the timer 31. The signal corresponding to the count time of the timer 82 delivered from the timer 82 represents the coagulation time, and thus a detection of the blood coagulation time is attained.

Towards the end of the coagulation reaction, the production of fibrin is terminated, and the rate of elevation of the temperature of the thermistor 2 is reduced, and accordingly, the temperature of the substance in the reaction vessel 3 tends to become a newly fixed temperature $T_4$ of, for example 70°C.

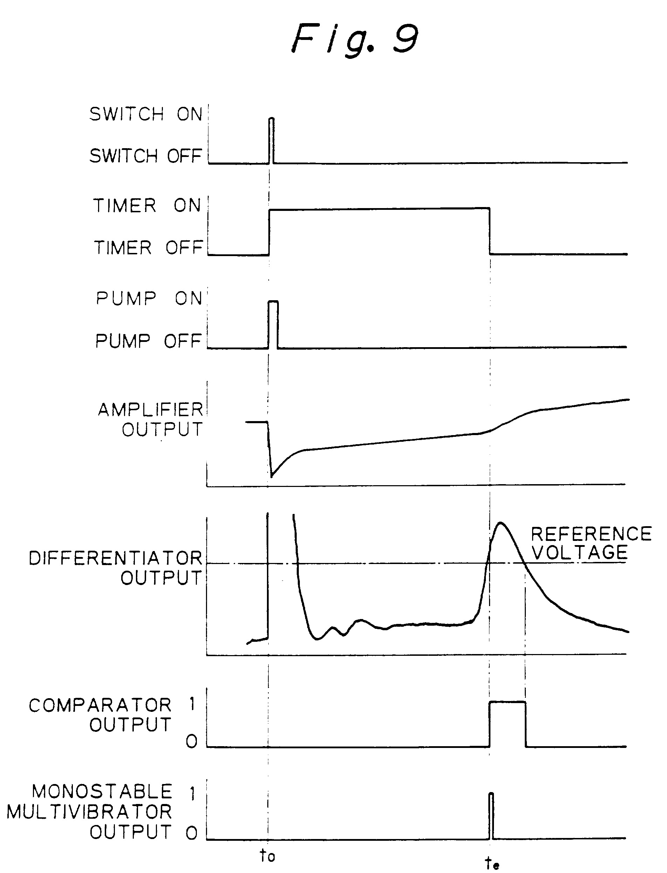

An example of the manner of detection of blood coagulation end point will be explained with reference to Fig. 9. Upon pressing of start switch 83, the operation of timer 82 and pump driver 91 are started (Fig. 9, 1st row). The counting of timer 82 is started (Fig. 9, 2nd row), the driving of pump 92 for injecting coagulation trigger reagent by pump driver 91 is started (Fig. 9, 3rd row), and the coagulation reaction is started with the injection of coagulation trigger reagent into blood sample 1 in reaction vessel 3.

The output of semiconductor sensor 2 during the process of blood coagulation reaction is amplified by amplifier 711 to provide the amplifier output (Fig. 9, 4th row). The output of amplifier 711 is differentiated with respect to time by differentiator 712 to provide the differentiator output in which a peak appears around at time of end of blood coagulation (Fig. 9, 5th row).

The output of differentiator 712 is compared in comparator 713 with the output voltage of potentiometer 714 as a reference voltage to provide the comparator output (Fig. 9, 5th and 6th rows). The output of comparator 713 is supplied to monostable multivibrator 81 which delivers pulse signal at the leading edge of the waveform of the output of comparator 713. The time $t_e$ of the delivered pulse signal is the detected blood coagulation end point $t_e$ (Fig. 9, 7th row).

The output of monostable multivibrator 81 is supplied to timer 82 to stop the counting of timer 82. The count of timer 82 at this stoppage represents the blood coagulation time.

For preventing erroneous operations in the transient period of the injection of the coagulation trigger reagent, it is possible to provide a gate circuit for preventing timer 82 from being stopped for a predetermined time of, for example, a few seconds after the start of the injection of the coagulation trigger reagent.

Instead of the above-described example of the manner of detection, alternative examples of the manner of detection, such as disclosed in USP 4217107, may be used.

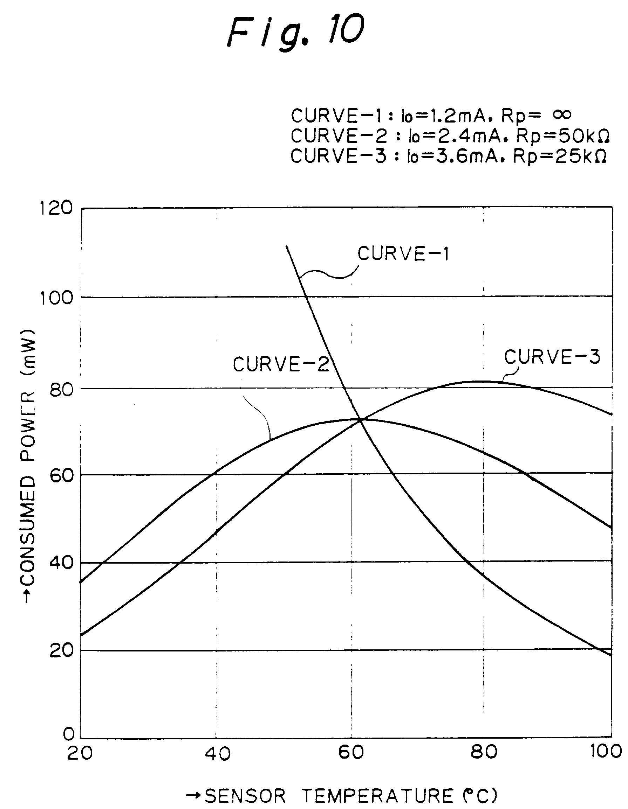

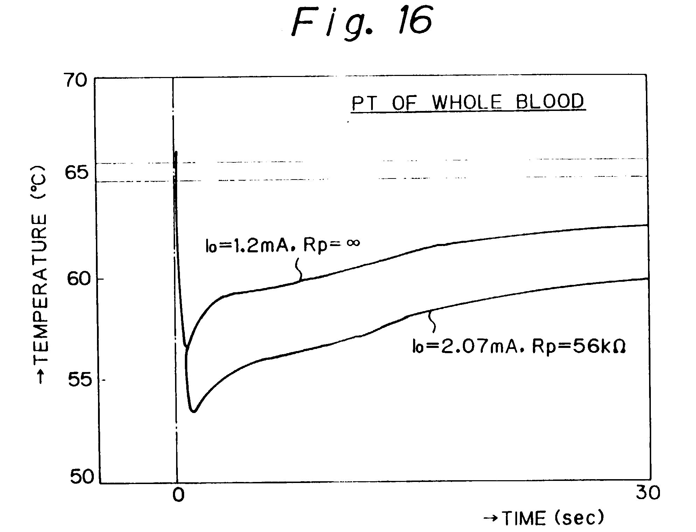

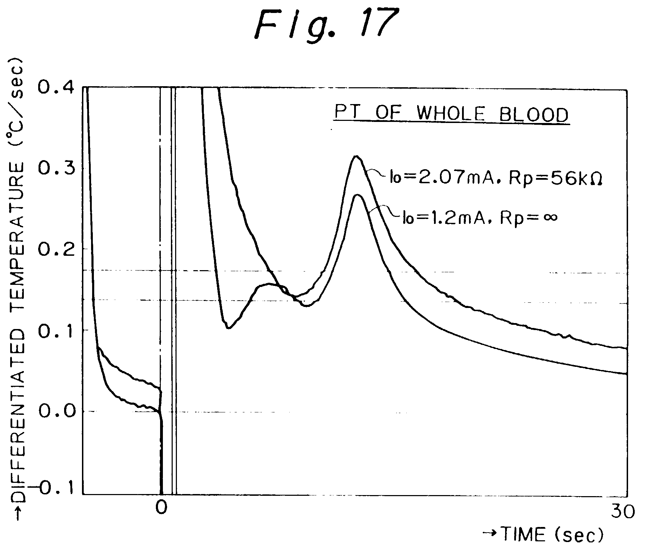

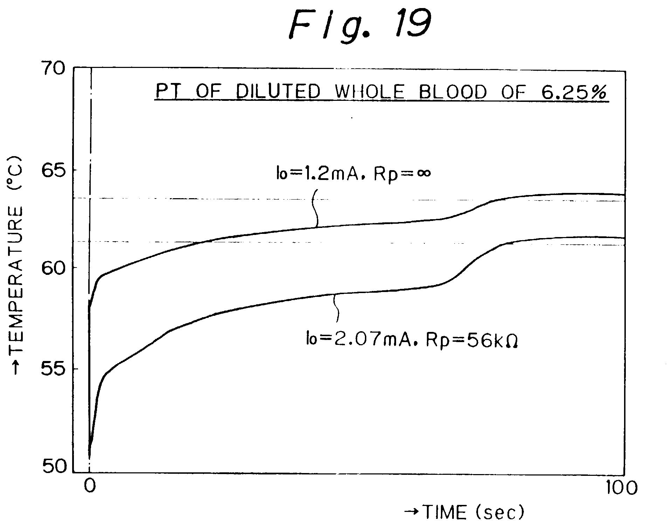

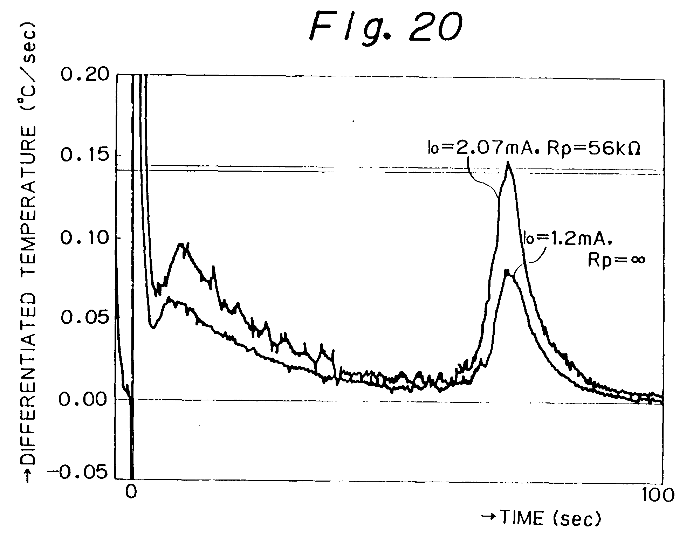

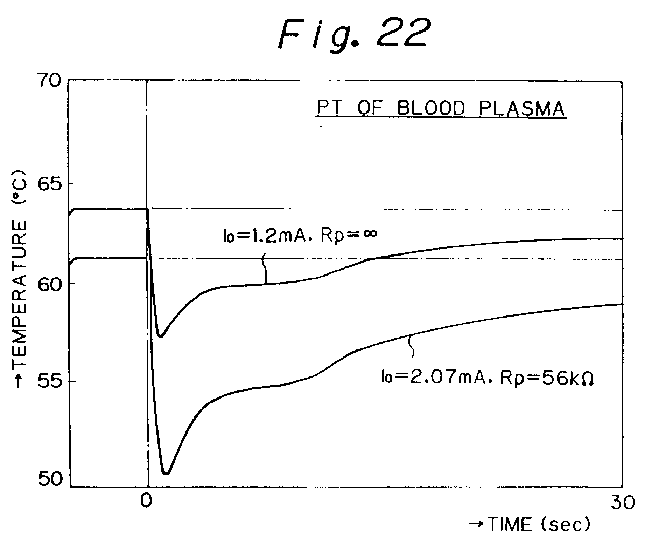

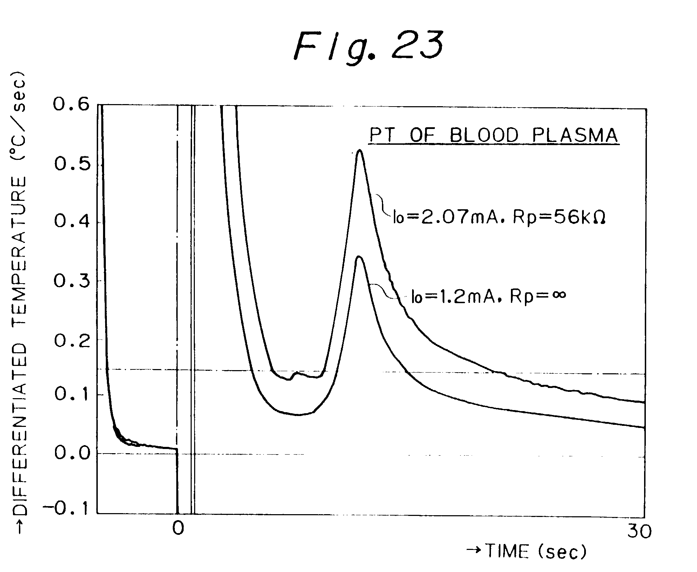

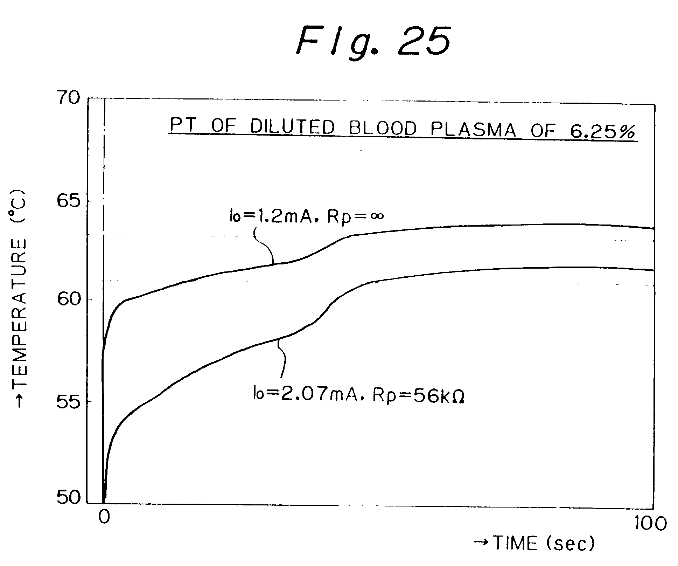

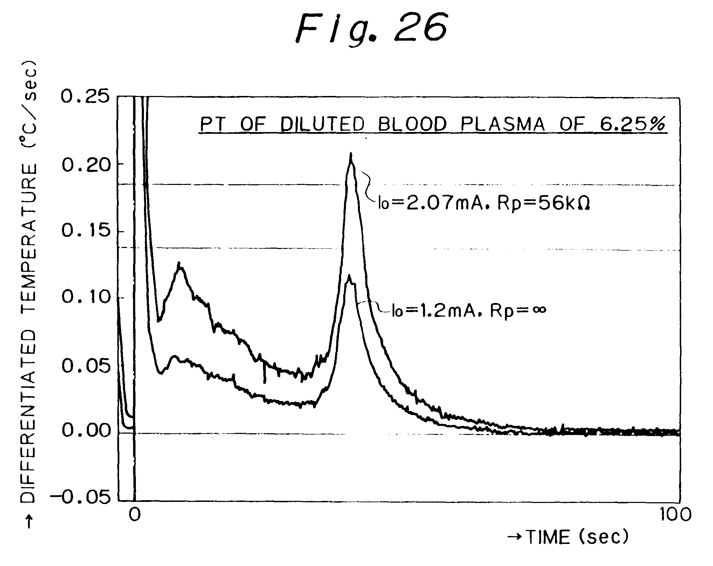

The characteristics of the operations of the apparatuses for measuring a blood coagulation time according to the embodiments of the present invention will be explained with reference to Figs. 10 to 31.

In Fig. 10, a characteristic of the relationship between the temperature of the thermistor as the semiconductor sensor and the power consumption is illustrated. This characteristic is derived from the result of a simulation by using a computer. The abscissa represents the temperature in °C of the thermistor, and the ordinate represents the consumed electric power in mW.

Curve-1 shows the case where the constant current $I_o$ is 1.2 mA, and the parallel resistor does not exist, i.e., the parallel resistance $R_p$ is infinite;

Curve-2 shows the case where the constant current $I_o$ is 2.4 mA, and the parallel resistance $R_p$ is 50 kΩ; and

Curve-3 shows the case where the constant current

$I_o$ is 3.6 mA, and the parallel resistance $R_p$ is 25 kΩ. The temperature corresponding to the end of the coagulation process is around 60°C.

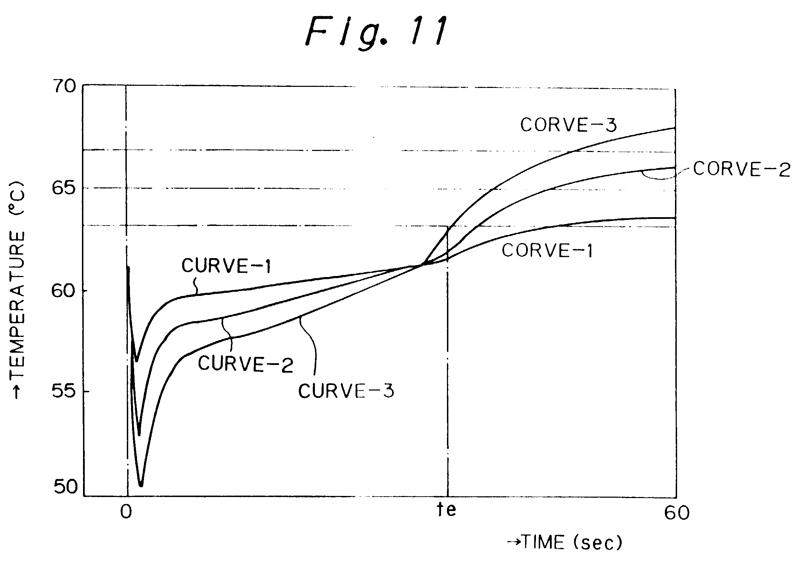

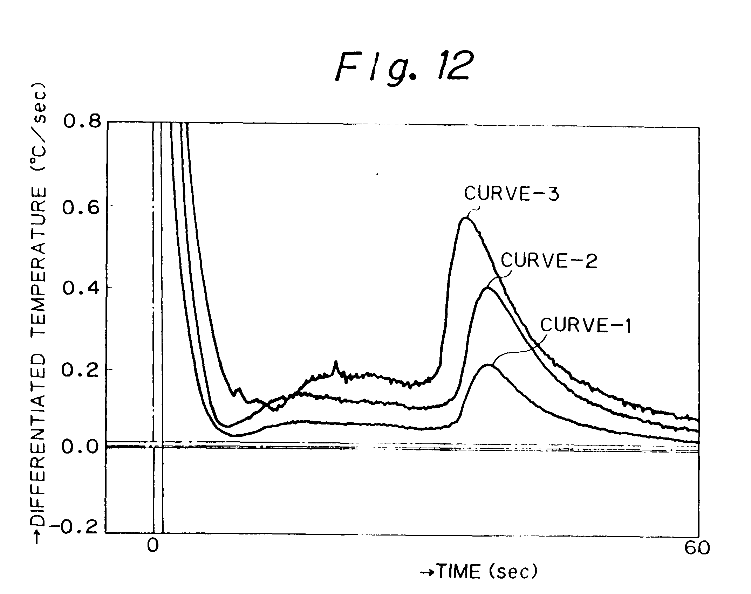

As the heat resistivity of blood sample is increased in the process of blood coagulation reaction, the temperature of the semiconductor sensor is raised. Assuming in Fig. 10 that the temperature of the semiconductor sensor at the end of blood coagulation is approximately 62°C, Curve-3 represents the fact that, as the temperature of the semiconductor sensor is raised, the electric power consumed by the semiconductor sensor is increased, that, as the electric power consumed by the semiconductor sensor is increased, the temperature of the semiconductor sensor is further raised, and that, in accordance with the rise of temperature of the semiconductor sensor, the electric power consumed by the semiconductor sensor is further increased. Based on this fact, an increase of the sensitivity of the semiconductor sensor can be expected due to the self positive feedback characteristic which will be seen from the characteristics shown in Figs. 11, 12, and so on.

In the characteristic shown in Fig. 10, the positive feedback does not take place over the peak of Curve-3, e.g. approximately 80°C, the thermal runaway of the semiconductor sensor can be prevented.

In Fig. 11, a characteristic of the process of the coagulation represented by a change of the temperature is illustrated. This characteristic is derived from the result of an experiment using the apparatus. The conditions of CURVE-1, CURVE-2, and CURVE-3 are the same as those in the case of Fig. 10. The end of the coagulation process occurs around a time $t_e$.

In Fig. 11, it is seen that, since, during the process of blood coagulation process from time $t_o$ when coagulation reagent is injected to time $t_e$ when blood coagulation is terminated, the temperature of the semiconductor sensor is lowest in Curve-3, and, after the detection of blood coagulation end point, the temperature of the semiconductor sensor becomes higher, the temperature of the semiconductor sensor affects less influences on the blood coagulation reaction, and that, since the difference in temperature becomes greatest around the blood coagulation end point, the sensitivity of temperature detection becomes greatest around the blood coagulation end points, which is advantageous for the measurement of the blood coagulation time.

In Fig. 12, a characteristic of the process of the coagulation represented by a change with time of the once differentiated temperature

$$\frac{dT}{dt} \ (°C/sec)$$

is illustrated. Curve-1, Curve-2, and Curve-3 correspond to those in the case of Fig. 11. The amount of change in the vicinity of the end of the coagulation process in Curve-3 is approximately 2.5 times that in Curve-1.

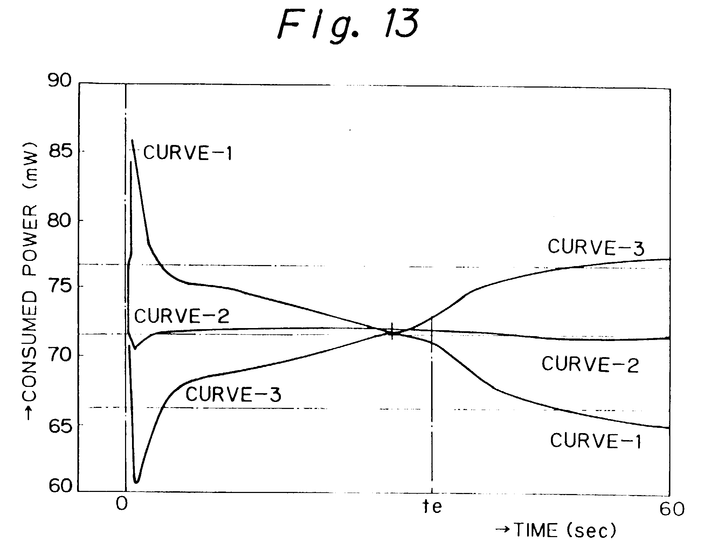

In Fig. 13, a characteristic of the process of the coagulation represented by a change with time of the power consumption is illustrated. The characteristic is derived from the result of an experiment using the apparatus.

In Fig. 13, as inferred from Curve-3 of Fig. 9, the electric power consumed by the semiconductor sensor is low before the blood coagulation end point $t_e$ and becomes higher after $t_e$ in Curve-3 of Fig. 13, which supports the characteristic shown in Fig. 11.

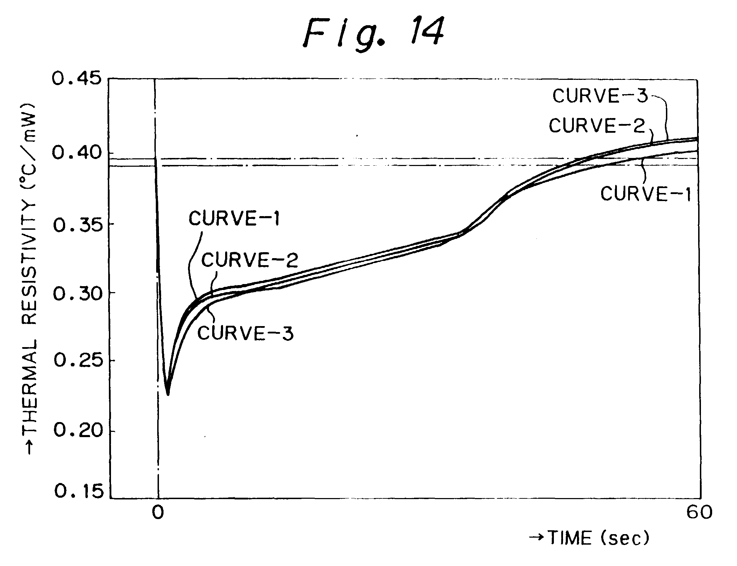

In Fig. 14, a characteristic of the process of the coagulation represented by a change with time of the thermal resistivity is illustrated. The characteristic is derived from the result of an experiment using the apparatus.

In Fig. 14, the temperature of the semiconductor sensor in Fig. 11, the differentiated temperature of the semiconductor sensor in Fig. 12, and the electric power consumed by the semiconductor sensor are expressed in thermal resistivity as results of normalization, and Curves-1, -2, and -3 approximately coincide, which represents the fact that the influence from the operating condition of the semiconductor sensor is reduced.

Being used in the conditions of CURVE-3 in Figs. 10, 11, 12, and 13, the semiconductor sensor reveals a high sensitivity, and a better signal to noise ratio, and affects less influences on the measurement, which provides the most suitable means for measuring the blood coagulation time.

If the blood coagulation end point is measured by using the curve of thermal resistivity versus time shown in Fig. 14 while the semiconductor sensor is operated under such the most suitable condition, the influences affected from the operating condition of the semiconductor sensor are reduced, and thereby a stable measurement of the blood coagulation time becomes possible.

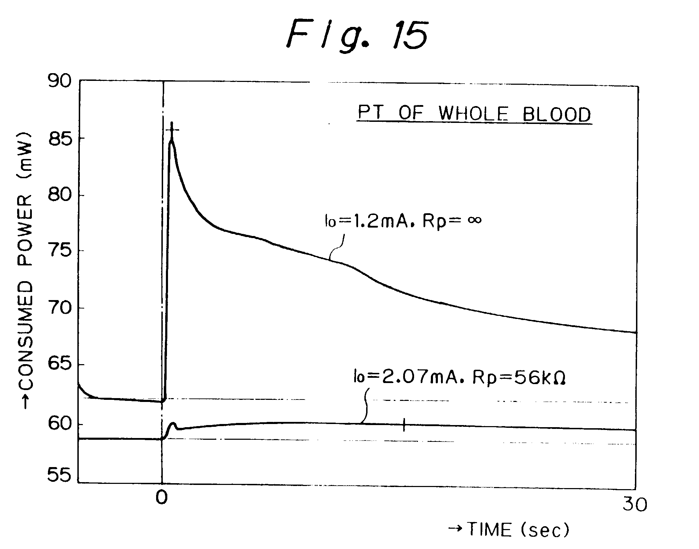

In Figs. 15, 16, and 17, the PT characteristics for whole blood represented by the electric power, the temperature, and the once-differentiated temperature

$$\frac{dT}{dt} \ (°C/sec)$$

are illustrated. The characteristics are derived from the results of experiments using the apparatus. The condition of the constant current $I_o$ and the parallel resistance $R_p$ is $I_o$ = 2.07 mA, $R_p$ = 56 kΩ or $I_o$ = 1.2 mA, $R_p = \infty$.

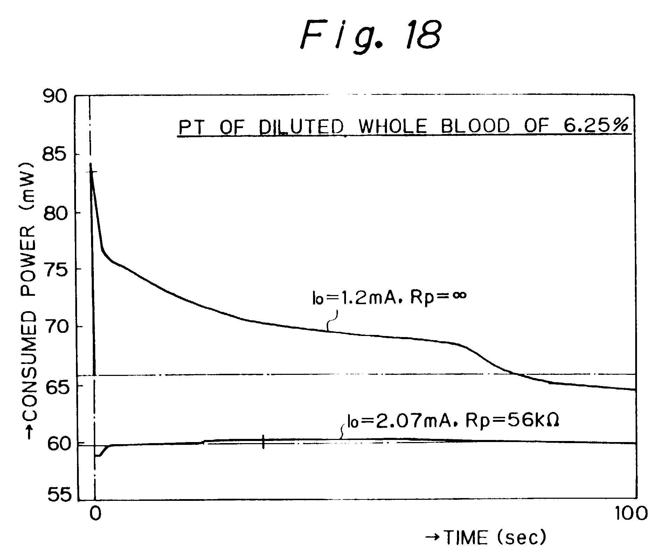

In Figs. 18, 19, and 20, the PT characteristics for diluted whole blood of 6.25% are illustrated.

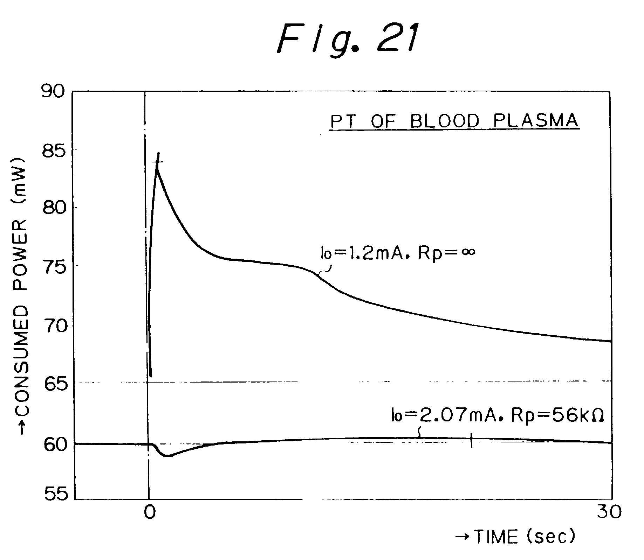

In Figs. 21, 22, and 23, the PT characteristics for blood plasma are illustrated.

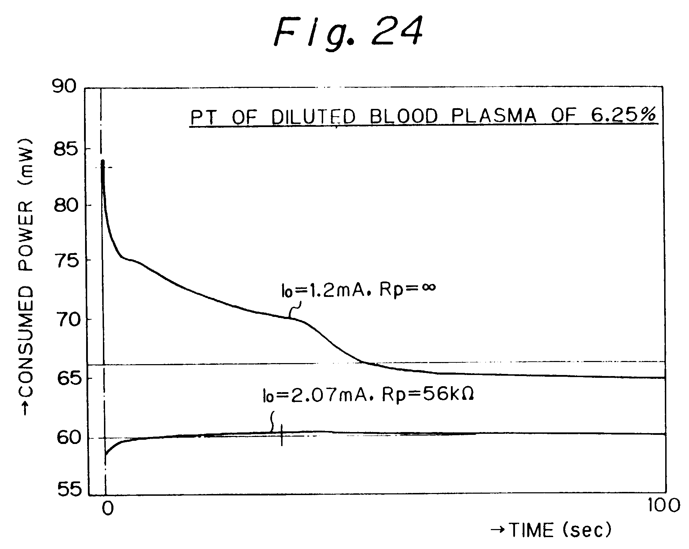

In Figs. 24, 25, and 26, the PT characteristics for diluted blood plasma of 6.25% are illustrated.

The operating conditions of the semiconductor sensor shown in Figs. 15 to 26, which is the intermediate of CURVE-1 and CURVE-2 of Fig. 13, may not be the best operating condition. Nevertheless, good patterns of the blood coagulation reaction process for the concentration 100% and 6.25% of each of whole blood and blood plasma are obtained as shown in Figs. 15 to 26.

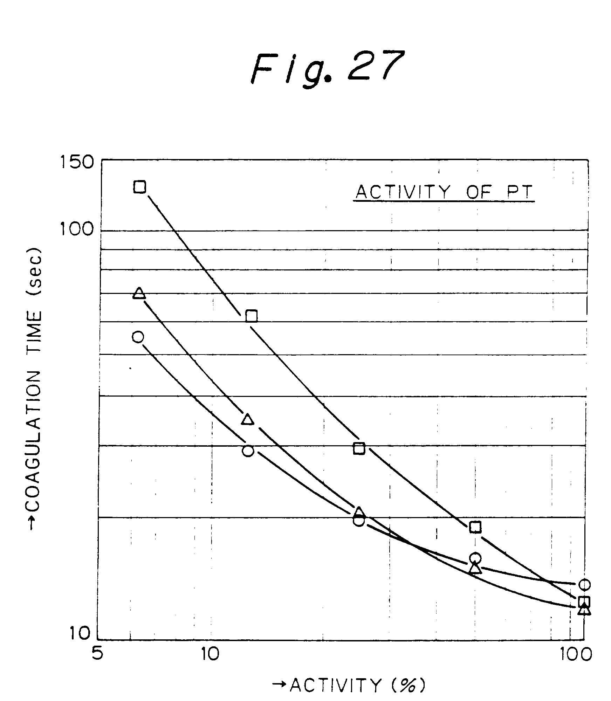

In Fig. 27, a characteristic of the PT activity is illustrated. The characteristic is derived from the result of an experiment using the apparatus.

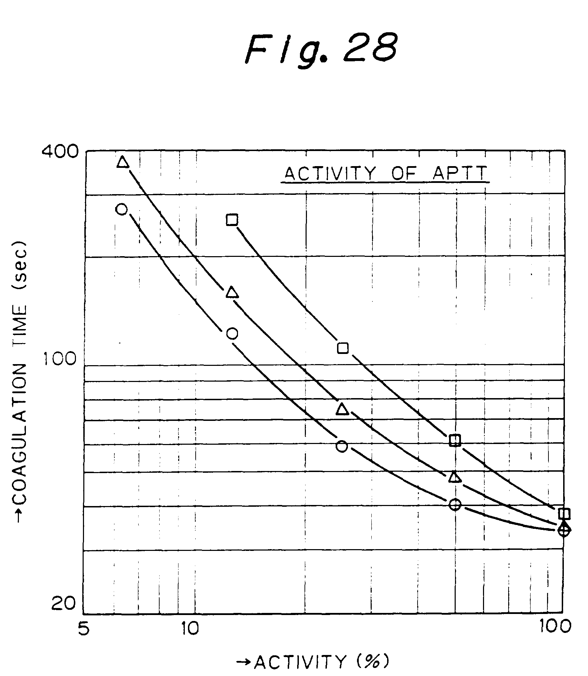

In Fig. 28, a characteristic of the APTT activity is illustrated.

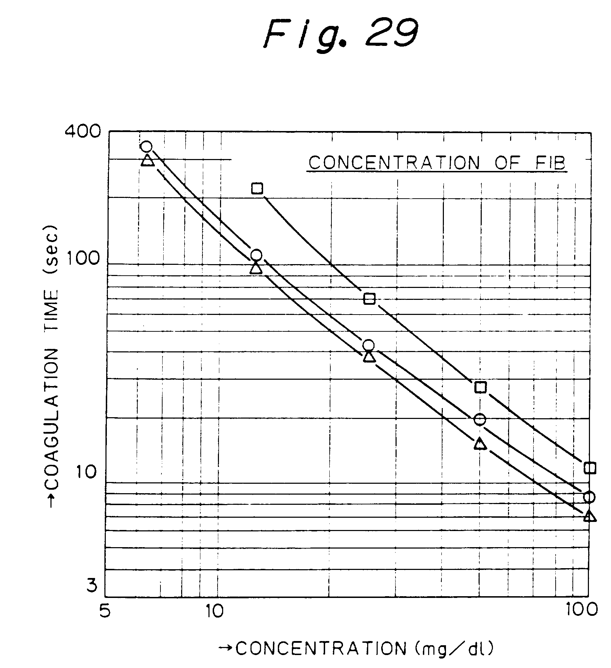

In Fig. 29, a characteristic of the FIB concentration is illustrated.

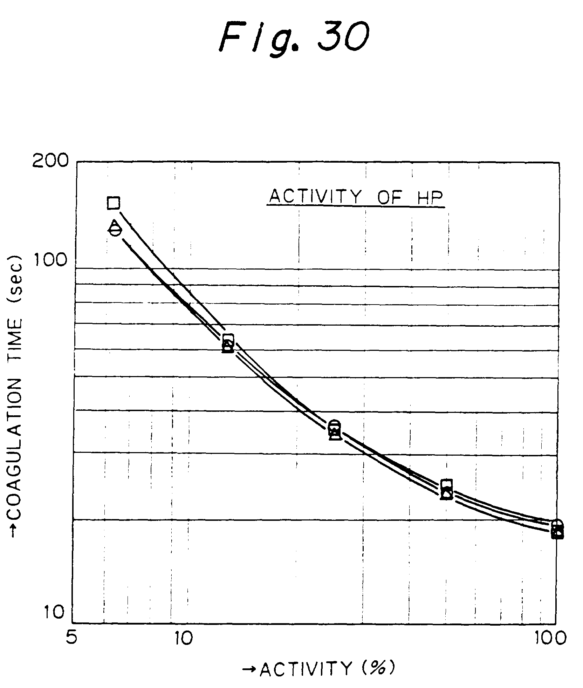

In Fig. 30, a characteristic of the HP activity is illustrated.

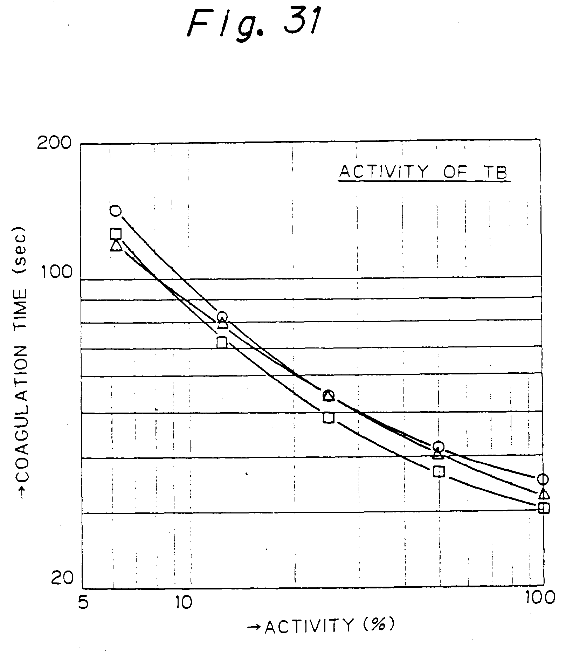

In Fig. 31, a characteristic of the TB activity is illustrated.

In the characteristics shown in Figs. 27 to 31, the characteristics of normal coagulation control for monitoring coagulation tests (VERIFY NORMAL CITRATE, ORGANON TEKNIKA CORPORATION) are represented by curves with plotting of circle mark, the characteristics of fresh blood plasma are represented by curves with plotting of triangle mark, and the characteristics of fresh whole blood are represented by curves with plotting of square mark.

It can be seen that, in the characteristics illustrated in Figs. 27 to 31, high precision measurements can be attained for an activity of from less than 10% to 100%.

It should be noted that the stable measurement for the activity of 6.25% for APTT shown in Fig. 28 is impossible in prior art method of measurement.

Although only examples of the apparatus according to the embodiments of the present invention are described, various modifications can be applied to the described examples. For example, for the semiconductor sensor, elements or circuits having a negative temperature coefficient of resistance, other than a thermistor, can be used, and for example, elements such as a diode or transistor, or circuits in which such elements are combined, can be used. Also, instead of a power source circuit having the external resistor, a power source circuit in which an internal resistance is included can be used. Also, instead of a direct current type power source, an alternate current type power source can be used.

**Claims**

1. An apparatus for measuring a blood coagulation time by a measurement of a period from a time of an addition of a coagulation reagent to a sample of blood, such as blood plasma and whole blood, to a time of a coagulation of the sample, said apparatus comprising:

   a vessel (3) for holding a sample of blood (1) such as blood plasma or whole blood;
   a semiconductor sensor (2) having a negative temperature coefficient and being immersed in the sample of blood held in said vessel;
   an electric power source (5,5') connected to said semiconductor sensor;
   a positive self-feedback resistor (6,6') connected in association with said power source for increasing the sensitivity of detection; and
   a detection circuit (71,71') connected to the output terminals of said semiconductor sensor (2) for detecting output signals of said semiconductor sensor.

2. An apparatus according to claim 1, wherein said electric power source (5) is a constant current power source.

3. An apparatus according to claim 2, wherein said resistor (6) is connected in parallel in the output circuit of said power source.

4. An apparatus according to claim 2, wherein said resistor is constituted by an internal resistance of said power source.

5. An apparatus according to claim 1, wherein said electric power source (5') is a constant voltage power source.

6. An apparatus according to claim 5, wherein said resistor (6') is connected in series in the output circuit of said power source.

7. An apparatus according to claim 5, wherein said resistor is constituted by an internal resistance of said power source.

8. An apparatus according to claim 1, wherein said apparatus further comprises an injection pump (92) for injecting a coagulation reagent into said vessel (3), an injection pump driver (91) for driving said injection pump, a start switch (83) connected to said injection pump driver for controlling the start of said injection pump driver, and a timer (82) responsive to the operations of said start switch and said detection circuit (71) for delivering an output signal as an indication of a blood coagulation time.

**Patentansprüche**

1. Vorrichtung zur Messung einer Blutgerinnungszeit durch Messung eines Zeitintervalls ab einem Zeitpunkt einer Gerinnungsreagenzbeimengung zu einer Blutprobe, wie Blutplasma und Vollblut, bis zu einem Gerinnungszeitpunkt der Probe, umfassend:

   ein Gefäß (3) zur Aufnahme einer Blutprobe (1) wie Blutplasma oder Vollblut;
   einen Halbleitersensor (2), der einen negativen Temperaturkoeffizienten aufweist und in die in dem Gefäß befindliche Blutprobe eingetaucht ist;
   eine elektrische Energiequelle (5, 5'), die mit dem Halbleitersensor verbunden ist;
   einen selbstrückkoppelnden Widerstand (6, 6') zur Erhöhung der Erfassungsempfindlichkeit, der in Verbindung mit der Energiequelle angeschlossen ist; und

eine Erfassungsschaltung (71, 71') zur Erfassung der Ausgangssignale des Halbleitersensors, die an den Ausgangsklemmen des Halbleitersensors (2) angeschlossen ist.

2. Vorrichtung gemäß Anspruch 1, wobei die elektrische Energiequelle (5) eine Konstantstrom-Energiequelle ist.

3. Vorrichtung gemäß Anspruch 2, wobei der Widerstand (6) in der Ausgangsschaltung der Energiequelle parallelgeschaltet ist.

4. Vorrichtung gemäß Anspruch 2, wobei der Widerstand aus einem Innenwiderstand der Energiequelle besteht.

5. Vorrichtung gemäß Anspruch 1, wobei die elektrische Energiequelle (5') eine Konstantspannungsenergiequelle ist.

6. Vorrichtung gemäß Anspruch 5, wobei der Widerstand (6') in der Ausgangsschaltung der Energiequelle in Reihe geschaltet ist.

7. Vorrichtung gemäß Anspruch 5, wobei der Widerstand aus einem Innenwiderstand der Energiequelle besteht.

8. Vorrichtung gemäß Anspruch 1, ferner umfassend:

eine Injektionspumpe (92) zum Injizieren eines Gerinnungsreagenz in das Gefäß (3),
einen Injektionspumpentreiber (91) zum Antreiben der Injektionspumpe,
einen Anlaßschalter (83) zur Steuerung der Betriebsaufnahme der Injektionspumpe, der an dem Injektionspumpentreiber angeschlossen ist, und
eine Uhr zur Abgabe eines Ausgangssignals als Anzeige der Blutgerinnungszeit, die auf den Betrieb des Anlaßschalters und der Erfassungsschaltung (71) reagiert.

**Revendications**

1. Appareil de mesure du temps de coagulation du sang par mesure du temps écoulé entre le moment de l'addition d'un réactif de coagulation à un échantillon sanguin, tel que du plasma sanguin et du sang total, et le moment de la coagulation de l'échantillon, ledit appareil comprenant :

un récipient (3) pour recueillir un échantillon de sang (1) tel que du plasma sanguin ou du sang total ;

un capteur à semi-conducteur (2) ayant un coefficient de température négatif et étant immergé dans l'échantillon de sang contenu dans ledit récipient ;
une source de courant électrique (5, 5') reliée audit capteur à semi-conducteur ;
une résistance à auto-régulation positive (6, 6') reliée en association avec ladite source de courant électrique pour augmenter la sensibilité de la détection ; et
un circuit de détection (71, 71') relié aux bornes de sortie dudit capteur à semi-conducteur (2) pour détecter des signaux de sortie dudit capteur à semi-conducteur.

2. Appareil selon la revendication 1, dans lequel ladite source de courant électrique (5) est une source de courant continu.

3. Appareil selon la revendication 2, dans lequel ladite résistance (6) est reliée en parallèle au circuit de sortie de ladite source de courant.

4. Appareil selon la revendication 2, dans lequel ladite résistance est constituée par une résistance interne de ladite source de courant.

5. Appareil selon la revendication 1, dans lequel ladite source de courant (5') est une source de courant à tension constante.

6. Appareil selon la revendication 5, dans lequel ladite résistance (6') est reliée en série dans le circuit de sortie de ladite source de courant.

7. Appareil selon la revendication 5, dans lequel ladite résistance est constituée par une résistance interne de ladite source de courant.

8. Appareil selon la revendication 1, dans lequel ledit appareil comprend en outre une pompe à injection (92) pour l'injection d'un réactif de coagulation dans ledit récipient (3), une commande de pompe à injection (91) pour commander ladite pompe à injection, un interrupteur de marche (83) relié à ladite commande de pompe à injection pour contrôler le déclenchement de ladite commande de pompe à injection, et un chronomètre (82) sensible au fonctionnement dudit interrupteur de marche et dudit circuit de détection (71) pour délivrer un signal de sortie servant d'indication du temps de coagulation du sang.

# Fig. 1

CONSTANT CURRENT POWER SOURCE

VOLTAGE DETECTION PORTION

VOLTAGE DETECTION CIRCUIT

METER

RECORDER

AMPLIFIER

6 EXTERNAL PARALLEL RESISTOR (Rₚ)

EP 0 476 923 B1

Fig. 2

TEMPERATURE OF THERMISTOR

$T_1$

$t_{on}$ $t_0$

$T_2$

$T_3$

$\rightarrow$ TIME

# Fig. 3

# Fig. 4

EP 0 476 923 B1

70

SENSOR OUTPUT DETECTION DEVICE

INPUT → | AC AMPLIFIER | → | BAND PASS FILTER | → | PHASE DETECTOR | → | LOW PASS FILTER | → OUTPUT

701     702     703     704

# Fig. 5

```
        5                                          71″
┌──────────────┐                          ┌──────────────┐
│ CONSTANT     │                          │ CURRENT      │
│ CURRENT      │                          │ DETECTION    │
│ POWER        │                          │ PORTION      │
│ SOURCE       │                          │              │
└──────────────┘     Rₚ                   └──────────────┘
                    ─\/\/─

              ┌──────────────┐
              │ THERMISTOR   │  2
              └──────────────┘
```

# Fig. 6

```
        5′              Rₛ                       71″
┌──────────────┐      ─\/\/─              ┌──────────────┐
│ CONSTANT     │                          │ CURRENT      │
│ VOLTAGE      │                          │ DETECTION    │
│ POWER        │                          │ PORTION      │
│ SOURCE       │                          │              │
└──────────────┘                          └──────────────┘

              ┌──────────────┐
              │ THERMISTOR   │  2
              └──────────────┘
```

# Fig. 7

POWER SOURCE

51

52

5

53 AMPLIFIER

6

PUMP
DRIVER
91

PUMP
92

4
2
1
3

VOLTAGE
DETECTION
PORTION

711 AMPLIFIER

712 DIFFEREN-
TIATOR

714

713 COMPARATOR

71

81 MONOSTABLE
MULTI-
VIBRATOR

TIMER
82

83 START
SWITCH

SIGNAL
REPRESENTING
COAGULATION
TIME

# Fig. 8

# Fig. 9

SWITCH ON
SWITCH OFF

TIMER ON
TIMER OFF

PUMP ON
PUMP OFF

AMPLIFIER
OUTPUT

DIFFERENTIATOR
OUTPUT

REFERENCE
VOLTAGE

COMPARATOR 1
OUTPUT 0

MONOSTABLE 1
MULTIVIBRATOR
OUTPUT 0

$t_o$                    $t_e$

# Fig. 10

CURVE-1 : Io=1.2mA, Rp= ∞
CURVE-2 : Io=2.4mA, Rp=50kΩ
CURVE-3 : Io=3.6mA, Rp=25kΩ

# Fig. 11

Fig. 12

# Fig. 13

Fig. 14

EP 0 476 923 B1

# F I g. 15

PT OF WHOLE BLOOD

Io=1.2mA. Rp=∞

Io=2.07mA. Rp=56kΩ

→CONSUMED POWER (mW)

→TIME (sec)

*Fig. 16*

EP 0 476 923 B1

Fig. 17

EP 0 476 923 B1

## Fig. 18

PT OF DILUTED WHOLE BLOOD OF 6.25%

Io=1.2mA, Rp=∞

Io=2.07mA, Rp=56kΩ

→CONSUMED POWER (mW)

→TIME (sec)

## Fig. 19

PT OF DILUTED WHOLE BLOOD OF 6.25%

$I_0 = 1.2mA$, $R_p = \infty$

$I_0 = 2.07mA$, $R_p = 56k\Omega$

→TEMPERATURE (°C)

→TIME (sec)

EP 0 476 923 B1

## Fig. 20

Graph: →DIFFERENTIATED TEMPERATURE (°C/sec) vs →TIME (sec)

Y-axis values: 0.20, 0.15, 0.10, 0.05, 0.00, -0.05

X-axis values: 0, 100

Io=2.07mA, Rp=56kΩ

Io=1.2mA, Rp=∞

EP 0 476 923 B1

Fig. 21

PT OF BLOOD PLASMA

$I_0=1.2mA. Rp=\infty$

$I_0=2.07mA. Rp=56k\Omega$

→CONSUMED POWER (mW)

→TIME (sec)

Fig. 22

PT OF BLOOD PLASMA

$I_0=1.2mA$, $Rp=\infty$

$I_0=2.07mA$, $Rp=56k\Omega$

→TIME (sec)

→TEMPERATURE (°C)

EP 0 476 923 B1

## F l g. 23

PT OF BLOOD PLASMA

$I_0=2.07mA$, $R_P=56k\Omega$

$I_0=1.2mA$, $R_P=\infty$

→DIFFERENTIATED TEMPERATURE (°C/sec)

→TIME (sec)

EP 0 476 923 B1

EP 0 476 923 B1

# Fig. 24

PT OF DILUTED BLOOD PLASMA OF 6.25%

$I_0$=1.2mA. $R_p$=∞

$I_0$=2.07mA. $R_p$=56kΩ

→CONSUMED POWER (mW)

→TIME (sec)

Fig. 25

PT OF DILUTED BLOOD PLASMA OF 6.25%

Io=1.2mA, Rp=∞

Io=2.07mA, Rp=56kΩ

→TEMPERATURE (°C)

→TIME (sec)

EP 0 476 923 B1

Fig. 26

PT OF DILUTED BLOOD PLASMA OF 6.25%

$I_0 = 2.07$ mA, $R_p = 56$ kΩ

$I_0 = 1.2$ mA, $R_p = \infty$

DIFFERENTIATED TEMPERATURE (°C/sec)

→TIME (sec)

EP 0 476 923 B1

# Fig. 27

# Fig. 28

ACTIVITY OF APTT

→COAGULATION TIME (sec)

→ACTIVITY (%)

# Fig. 29

EP 0 476 923 B1

# Fig. 30

ACTIVITY OF HP

(y-axis) →COAGULATION TIME (sec)
(x-axis) →ACTIVITY (%)

# Fig. 31

ACTIVITY OF TB

→COAGULATION TIME (sec)

→ACTIVITY (%)